# DEMANDE DE BREVET EUROPEEN

(11) **EP 2 430 984 A1**
(43) Date de publication de la demande: **21.03.2012**
(21) Numéro de dépôt: 11181191.5
(22) Date de dépôt: 14.09.2011
(51) Int. Cl.: A61B 17/04

(54) **Composant d'implant de suture et dispositif d'implant de suture comprenant un tel composant**

(30) Priorité: 23.09.2010 FR 1057677; 16.09.2010 US 383665 P
(71) Demandeur: Tornier, Inc., Edina, MN 55435 (US)
(72) Inventeur: Peterson, David R., La Jolla, California 92037 (US); Ohashi, Kevin L., Jamaica PLain, Massachusetts 02130 (US); Schlotterback, Ryan D., Fort Wayne, Indiana 46845 (US)
(74) Mandataire: Grand, Guillaume

(57) **Abrégé**

La présente invention concerne un composant d'implant de suture (100), comprenant un corps (101) qui s'étend suivant un axe longitudinal (X100) et qui inclut au moins une ouverture (106) apte à recevoir un fil de suture (130), de sorte que le fil de suture coopérant avec corps forme sensiblement un U ou un V. Le composant est caractérisé en ce que le corps (101) est déformable, sous l'effet d'une tension exercée par le fil de suture (130) au moins sur une extrémité distale (103) du corps, entre une configuration de repos dans laquelle le corps (101) présente une première valeur de dimension longitudinale et une première valeur de dimension transversale, et une configuration active dans laquelle le corps (101) présente une deuxième valeur de dimension longitudinale (LA) qui est inférieure à la première valeur de dimension longitudinale et une deuxième valeur de dimension transversale (DA) qui est supérieure à la première valeur de dimension transversale. L'invention concerne également un dispositif d'implant de suture, comprenant un composant d'implant de suture (100) tel que mentionné ci-dessus, un fil de suture (130), et des moyens (131 a, 132a, 131 b, 132b, 133) de coopération du fil de suture avec le corps (101) du composant, situés au moins du côté de l'extrémité distale (103) du corps.

## Description

La présente invention concerne un composant d'implant de suture. L'invention concerne également un dispositif d'implant de suture comprenant un tel composant. Le domaine de l'invention est celui des implants de suture pour la fixation d'un tissu mou devant être réparé à un os, par exemple un muscle, un tendon, ou un ligament tel que le labrum glénoïdal.

De manière connue, le chirurgien dispose de différents dispositifs de ré-attachement du tissu mou à l'os en vue de sa guérison, telles que des vis, agrafes, punaises, clous ou même un fil de suture seul. Avantageusement, une cavité osseuse peut être préparée dans l'os du patient au point désiré de ré-attachement du tissu mou, et un implant de suture est alors ancré dans cette cavité pour permettre la fixation d'un fil de suture chirurgical. Un tel implant peut être en métal, polymère, ou bio-matériau résorbable. Egalement l'implant peut être fileté, inséré en force dans la cavité, ou bien bloqué après insertion.

Toutefois, les dispositifs connus de réparation du labrum glénoïdal et autres tissus mous présentent des difficultés de positionnement, ainsi que certains risques : perte osseuse ou ostéoporose, réaction avec les polymères, ou rupture de certains éléments, lesquels peuvent rester dans le corps du patient et gêner la guérison. Par ailleurs, le retrait de l'os est accru en raison du matériel qui y est implanté. Ainsi, la réparation chirurgicale doit être peu invasive, c'est-à-dire laisser le moins possible de matière dans le corps, et présenter une interface tissu-os importante.

A cet effet, US-A-2008/0065114 décrit un composant d'implant pour l'ancrage d'un fil de suture chirurgical dans une cavité osseuse. Le composant présente deux ouvertures d'extrémité au travers desquelles s'étendent les deux extrémités du fil. Le composant est replié en forme de U ou de V et positionné contre un élément rigide préalablement inséré dans la cavité osseuse. Lorsqu'une tension est appliquée sur le fil de suture, le composant se replie sur lui-même et est bloqué par l'élément rigide. Cependant, un tel composant n'est pas satisfaisant car il nécessite un élément rigide supplémentaire pour l'ancrage du fil de suture dans la cavité osseuse. Par ailleurs, l'ancrage est réalisé par écrasement du composant contre l'élément rigide, et non par adhérence du composant d'implant de suture dans la cavité osseuse.

WO-A-2007 005 394 décrit un autre composant d'implant de suture, comprenant un corps conformé comme une bande plate allongée. Le corps s'étend suivant un axe longitudinal et inclut plusieurs ouvertures traversantes recevant un fil de suture. Le corps ne comprend pas de paroi déformable en accordéon autour de l'axe longitudinal. Ainsi, le composant n'est pas adapté pour procurer un ancrage satisfaisant dans une cavité osseuse. En outre, le composant n'est pas adapté pour recevoir des dispositifs additionnels, pour sa rigidification ou pour l'accroche du fil de suture, dans un espace intérieur qui serait délimité par une telle paroi.

Le but de la présente invention est de proposer un composant d'implant de suture chirurgical simple à fabriquer et à utiliser, peu encombrant et peu invasif.

A cet effet, l'invention a pour objet un composant d'implant de suture tel que défini à la revendication 1.

Ainsi, l'invention permet d'obtenir un composant d'implant de suture compact, simple à mettre en oeuvre et n'utilisant aucun élément rigide invasif, notamment en matériau métallique ou polymère rigide. Entre la configuration de repos et la configuration active, le corps du composant d'implant forme des interférences dans la cavité osseuse, selon un effet « accordéon », en étant est plissé, cintré, écrasé et/ou contracté. Autrement dit, le corps tubulaire présente une réduction de longueur et une augmentation de largeur, c'est-à-dire de diamètre, lequel augmente jusqu'à atteindre le diamètre de la cavité osseuse. Le composant est alors ancré par adhérence entre sa paroi et la paroi de la cavité osseuse, quel que soient les propriétés de cette cavité osseuse. En pratique, le composant et le fil de suture associé présentent une résistance à la tension suffisante dans le cadre de la réparation de tissu mou. Par ailleurs, le fil de suture entre et sort par une même ouverture de sorte que la manipulation du fil est simple et pratique.

D'autres caractéristiques avantageuses de l'invention, prises isolément ou en combinaison, sont définies aux revendications 2 à 13.

L'invention a également pour objet un dispositif d'implant de suture, tel que défini à la revendication 14.

Avantageusement, le dispositif est tel que défini à la revendication 15.

En outre, une variante particulière du dispositif est définie à la revendication 16.

L'invention sera mieux comprise à la lecture de la description qui va suivre, donnée uniquement à titre d'exemple et faite en se référant aux dessins sur lesquels :
- la figure 1 est une vue en perspective d'un dispositif d'implant de suture conforme à l'invention, comprenant un composant d'implant de suture en configuration de repos ;
- la figure 2 est une vue à plus grande échelle du détail II à la figure 1 ;
- les figures 3 à 5 sont des coupes longitudinales du composant des figures 1 et 2, à différentes étapes de mise en oeuvre ;
- les figures 6 et 7 sont des coupes longitudinales d'un deuxième mode de réalisation d'un composant d'implant de suture conforme à l'invention, respectivement en configuration de repos et en configuration active ;
- les figures 8 et 9 sont des coupes longitudinales d'un troisième mode de réalisation d'un composant d'implant de suture conforme à l'invention, respectivement en configuration de repos et en configuration active ;
- la figure 10 est une coupe longitudinale d'un quatrième mode de réalisation d'un composant d'implant de suture conforme à l'invention, en configuration de repos ;
- la figure 11 est une coupe longitudinale d'un cinquième mode de réalisation d'un composant d'implant de suture conforme à l'invention, en configuration de repos ;
- la figure 12 est une coupe longitudinale d'un sixième mode de réalisation d'un composant d'implant de suture conforme à l'invention, en configuration de repos ; et
- les figures 13 et 14 sont des coupes longitudinales d'un septième mode de réalisation d'un composant d'implant de suture conforme à l'invention, respectivement en configuration de repos et en configuration active.

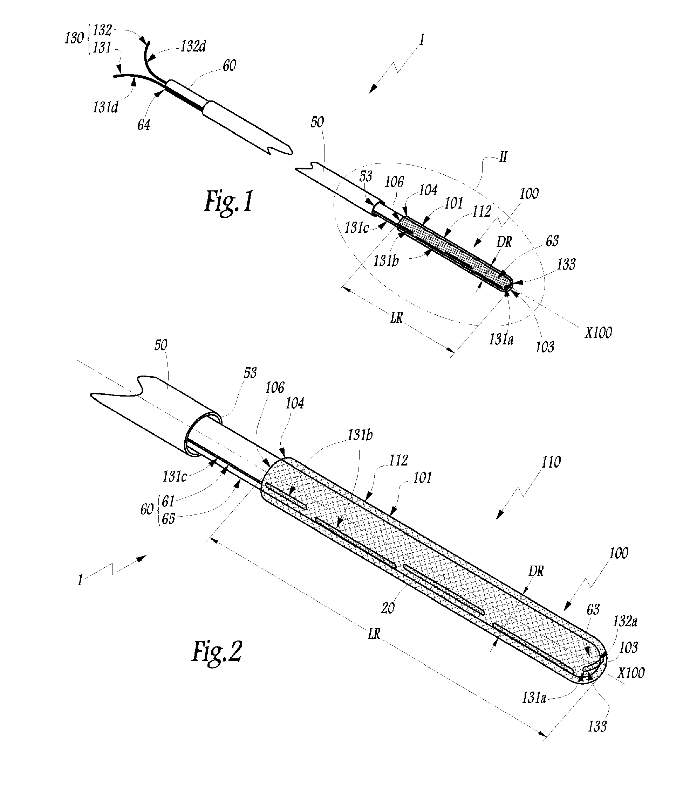

Sur les figures 1 à 5 est représenté un dispositif d'implant de suture 1, qui comprend un composant d'implant de suture 100. Le dispositif 1 comprend également un fil de suture 130, un manchon de compression 50 et une aiguille d'insertion 60, destinés à coopérer avec un corps 101 appartenant au composant 100.

Le manchon 50 et l'aiguille 60 constituent des moyens amovibles d'insertion du composant d'implant de suture 100 dans une cavité osseuse 81, préparée à cet effet par le chirurgien dans un os 80 du patient. L'aiguille 60 présente une forme de tige avec une extrémité distale 63, une extrémité proximale 64, une partie courante 65, ainsi que deux rainures 61 et 62 qui s'étendent longitudinalement dans la partie courante 65 entre les extrémités 63 et 64. Le manchon 50 présente une forme tubulaire et comprend une extrémité distale annulaire 53. En pratique, l'aiguille 60 est insérée de manière coulissante dans le manchon 50.

Le corps 101 du composant 100 présente une forme de tube allongé, avec une paroi déformable 112 qui s'étend selon un axe central X100 et délimite un espace intérieur 105. La paroi 112 est agencée tout autour de l'axe X100. Le corps 101 présente une extrémité distale 103 fermée, ainsi qu'une extrémité proximale 104 munie d'une ouverture 106.

En pratique, le corps 101 du composant 100 est fabriqué par tissage ou tressage, ou par toute autre technique adaptée. Des fils ou faisceaux de fils, fibres ou brins sont entrelacés pour former le corps 101. Les fibres utilisées peuvent être identiques, ou bien différents types de fibres peuvent présenter des matériaux ou dimensions variés. Par exemple, le corps 101 peut présenter au moins deux types de fibres ayant des diamètres respectifs différents. A titre de variantes non représentées certaines parties ou sections du corps 101 peuvent présenter une forme de filetage ou être rigidifiées afin de faire varier la flexibilité du corps 101, et ainsi favoriser sa déformation selon une géométrie particulière.

De préférence, le corps 101 et le fil 130 sont fabriqués en un matériau résorbable, de préférence en polyhydroxyalkanoate, en particulier en poly-4-hydroxybutyrate tel que du Tephaflex (marque déposée). Ainsi, le corps 101 et le fil 130 sont peu invasifs, tout en présentant des propriétés de résistance mécanique adaptées à la réparation de tissu mou.

En variante, le ou les matériaux suivants peuvent être employés pour le ou les différents types de fibres constituant le corps 101 du composant 100 et pour le fil 130 : polyester, polyéthylène, polyhydroxyalkanoate.

Le fil 130 est implanté dans le composant 100 en vue de l'opération chirurgicale. Le fil 130 présente deux brins 131 et 132 qui sont sensiblement rectilignes en position d'utilisation. Les brins 131 et 132 sont reliés par un arc 133 disposé à l'extrémité distale 103 du corps 101. Plus précisément, le brin 131 présente plusieurs parties longitudinales 131 a, 131 b, 131c et 131 d ayant chacune une fonction et un positionnement particuliers. Le brin 132 présente également plusieurs parties longitudinales 132a, 132b, 132c et 132d, qui sont similaires aux parties 131a à 131d du brin 131 et ne seront donc pas décrites en détail ci-après.

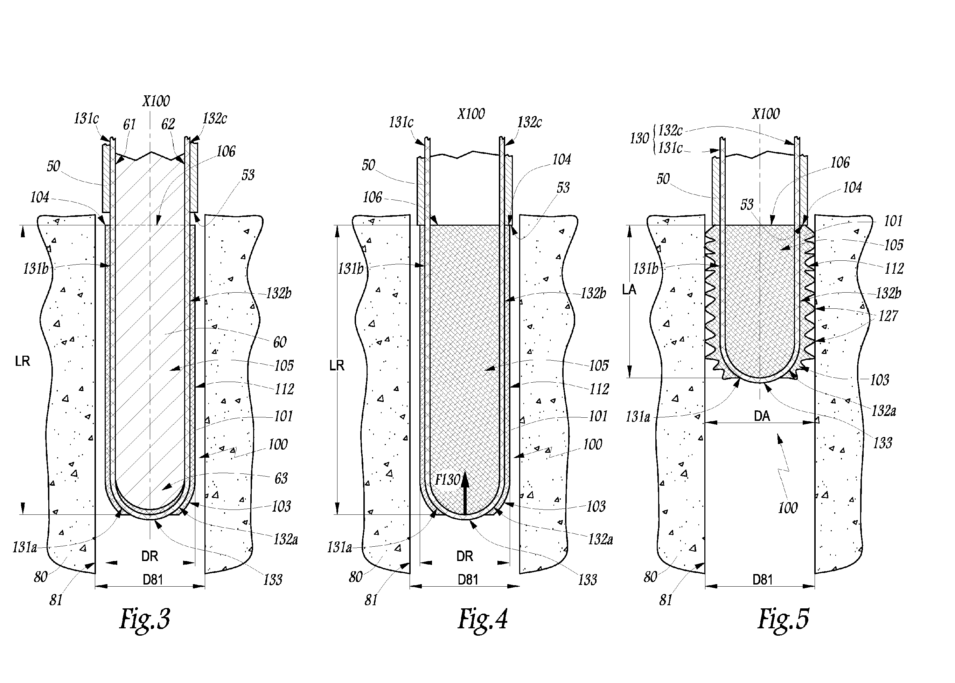

Dans la partie d'extrémité 131 a, le brin 131 passe à travers l'extrémité distale 103 du corps 101 pour rejoindre l'arc 133. Dans la partie 131 b qui s'étend entre l'extrémité distale 103 et l'extrémité proximale 104 du corps 101, le brin 131 est entrelacé avec le corps 101, comme visible sur les figures 1 et 2. Dans un but de simplification, cet entrelacement de la partie 131 b et du corps 101 n'est pas représenté sur les figures 3 à 5. Dans la partie 131c, le brin 131 est logé dans la rainure 61 de l'aiguille 60 afin de favoriser son maintien en position lors de l'insertion du composant 100 dans la cavité 81. Enfin, la partie 131 d émerge de la rainure 61 du côté de l'extrémité proximale 64 de l'aiguille 60.

Le fonctionnement du dispositif 1 est décrit ci-après en référence aux figures 1 à 5.

Tout d'abord, le fil 130 est implanté dans le composant 100 avant leur insertion dans la cavité osseuse 81. Grâce aux entrelacements des brins 131 et 132 et à l'arc 133, le fil 130 coopère mécaniquement avec le corps 101. Autrement dit, les parties 131 a et 131 b du brin 131, les parties 132a et 132b du brin 132, ainsi que l'arc 133 forment des moyens de coopération entre le fil 130 et le corps 101 du composant 100.

Ensuite, l'aiguille 60 est insérée dans le composant 100 par l'ouverture 106 qui est située à l'extrémité proximale 104. Ainsi, l'extrémité distale 63 de l'aiguille 60 est positionnée au niveau de l'extrémité distale 103 dans l'espace intérieur 105 du corps 101. Les deux brins 131 et 132 sont logés dans les rainures 61 et 62. Le manchon 50 est positionné sur l'aiguille 60 en coulissant dans sa partie courante 65. Le manchon 50 et l'aiguille 60 sont agencés coaxialement au composant 100.

A ce stade, le composant 100 se trouve dans une configuration de repos. Le corps présente une première valeur de dimension longitudinale LR et une première valeur de dimension transversale DR, étant noté que, en référence à l'axe X100, la dimension longitudinale correspond à la longueur du corps 101, tandis que la dimension transversale correspond au diamètre du corps 101.

Comme visible sur la figure 3, le chirurgien manipule le dispositif 1, et plus particulièrement l'aiguille 60, pour insérer le composant 100 en configuration de repos dans la cavité osseuse 81. Le diamètre DR du corps 101 est inférieur au diamètre D81 de la cavité 81. En raison de la présence de l'aiguille 60, dans cette configuration de repos, le corps 101 ne peut pas se déformer. Autrement dit, l'aiguille 60 permet de maintenir la stabilité de la dimension longitudinale LR et de la dimension transversale DR du corps 101.

Comme visible sur la figure 4, l'aiguille 60 est ensuite retirée du dispositif 1. L'aiguille 60 coulisse hors du composant 100 puis du manchon 50, tandis que les brins 131 et 132 glissent hors des rainures 61 et 62. A ce stade, le dispositif 1 peut être manipulé grâce au manchon 50 et aux parties 131 d et 132d du fil 130.

En pratique, une tension F130 est alors exercée sur le fil 130. Comme le fil 130 coopère avec le corps 101, et en particulier avec son extrémité 103, la tension F130 tend à extraire le composant 100 hors de la cavité 81. Cependant, le manchon 50 est adapté pour maintenir en position l'extrémité proximale 104 lors de la déformation du corps 101. Autrement dit, l'extrémité proximale 104 du corps 101 vient en butée contre l'extrémité distale 53 du manchon 50.

Ainsi, comme visible sur la figure 5, le corps 101 du composant 100 est comprimé contre le manchon 50 sous l'effet de la tension F130. Le corps 101 est déformé entre la configuration de repos et la configuration active, dans laquelle le corps 101 présente une deuxième valeur de dimension longitudinale LA qui est inférieure à la première valeur de dimension longitudinale LR et une deuxième valeur de dimension transversale DA qui est supérieure à la première valeur de dimension transversale DR. La paroi 112 initialement tubulaire se déforme, de sorte que dans la configuration active, la paroi 112 déformée est sensiblement bloquée en appui contre les parois de la cavité osseuse 81. En particulier, la déformation en accordéon de la paroi 112 autour de l'axe X100 forme plusieurs plis. La coopération entre les parties 131 b et 132b du fil 130 et la paroi 112 permet de déformer le corps 101 de manière contrôlée. Certains brins ou fibres tissés ou tressés constituant le corps 101 sont déformés en saillie de la paroi 112, formant ainsi des aspérités ou interférences 127 qui empêchent le mouvement relatif entre la cavité 81 et le composant 100. Autrement dit, la paroi 112 déformée présente plusieurs interférences 127 qui permettent d'ancrer le composant 100 dans la cavité osseuse 81, avec une adhérence par coopération mécanique de type coincement, frottement et/ou grippage.

A ce stade, le corps 101 est « contracté » dans la direction longitudinale LA et « dilaté » dans la direction transversale DA. Les interférences 127, générées par le plissage et/ou le cintrage contrôlé du corps 101 en « accordéon » dans la cavité 81, permettent au composant 100 de résister à la tension F130 qui tend à l'extraire de la cavité 81. Ainsi, le composant 100 et le fil 130 sont rigidement maintenus en position dans la cavité 81 en vue de la réparation de tissu mou.

Par ailleurs, le tissage ou tressage du corps 101 peut être optimisé pour favoriser la formation des interférences 127 et améliorer l'adhérence dans la cavité osseuse 81. Par exemple, en variante non représentée sur les figures 1 à 5, des brins ou fibres de tailles variables peuvent être utilisés dans le corps 101. Selon un autre exemple, un brin plus large peut être enroulé autour de la paroi 112 en configuration de repos, de manière analogue à un filetage extérieur. Les interférences 127 peuvent donc être radiales, régulièrement réparties sur la paroi 112, ou bien enroulées en spirale sur cette paroi 112. Ainsi, la fixation du composant 100 en configuration active, par adhérence de la paroi 112 dans la cavité 81, est facilitée et renforcée.

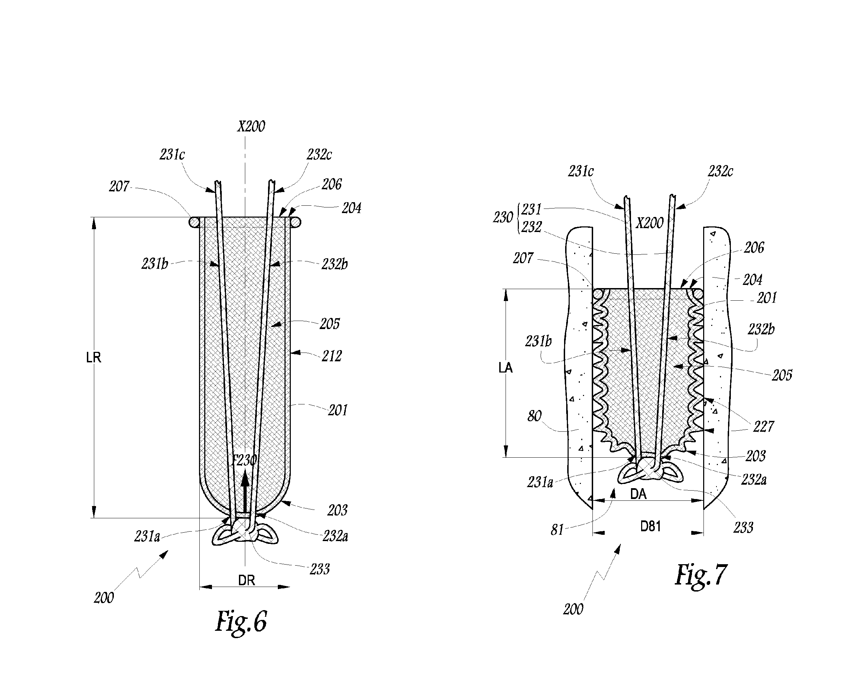

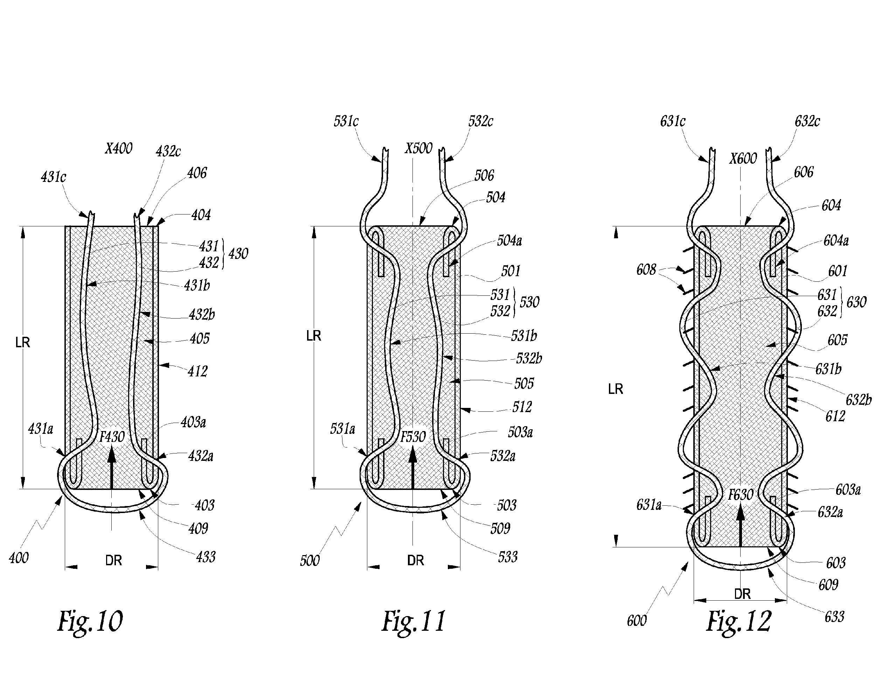

Sur les figures 6 à 14 sont représentés des composants d'implants de suture 200, 300, 400, 500, 600 et 700. De préférence, ces composants peuvent être mis en oeuvre avec un manchon 50 et une aiguille 60, non représentés sur les figures 6 à 14 mais similaires au premier mode de réalisation. Par ailleurs, la déformation de ces composants entre une configuration de repos et une configuration active, ainsi que l'ancrage de ces composants dans la cavité osseuse 81 en configuration active, sont comparables avec le fonctionnement du composant 100 selon le premier mode de réalisation. Dans un but de simplification, l'os 80 et la cavité 81 ne sont pas représentés sur les figures 10 à 14.

Sur les figures 6 et 7 est représenté le composant d'implant de suture 200.

Certains éléments constitutifs du composant 200 sont similaires aux éléments constitutifs du composant 100 du premier mode de réalisation, décrit plus haut, et portent la même référence augmentée de cent. Il s'agit de l'axe X200, du corps 201, de l'extrémité distale 203, de l'extrémité proximale 204, de l'ouverture 206, de l'espace intérieur 205, de la paroi 212, des dimensions DR et LR en configuration de repos, des dimensions DA et LA en configuration active, des aspérités 227, du fil 230, ainsi que des brins 231 et 232 qui présentent des parties de brins 231 a, 232a, 231 b, 232b, 231c et 232c.

Un anneau rigide 207 est attaché à l'extrémité proximale 204 du corps 201 et renforce la structure du composant 200. En pratique, les dimensions de l'anneau 207 correspondent aux dimensions de la cavité 81, afin que le composant 200 adhère aux parois de la cavité 81 dès son insertion dans cette cavité 81. En outre, l'anneau 207 est destiné à venir en butée contre l'extrémité distale 53 du manchon de compression 50. De ce fait, les risques de déformation ou de glissement incontrôlés de l'extrémité proximale 204 lors du passage de la configuration au repos à la configuration active sont réduits.

Par ailleurs, les brins 231 et 232 traversent l'extrémité distale 203, respectivement au niveau des parties 231 a et 232a, et sont reliés par un arc 233 pourvu d'un noeud qui est situé à l'extérieur de l'extrémité distale 203 du corps 201. Les parties 231 b et 232b s'étendent dans l'espace intérieur 205 du corps 201 et à travers l'ouverture 206 sans être entrelacées avec la paroi 212. Cette méthode d'implantation du fil 230 dans le composant 200 est plus rapide en comparaison avec le premier mode de réalisation. En outre, les brins 231 et 232 peuvent être initialement indépendants ou liés.

Ainsi, le fil 230 coopère avec le corps 201 du composant 200 par l'intermédiaire des parties 231 a et 232a et de l'arc 233 qui est noué. Une tension F230 peut être exercée sur l'extrémité distale 203 du corps 201 en manipulant le fil 230, déformant ainsi le corps 201 en configuration active.

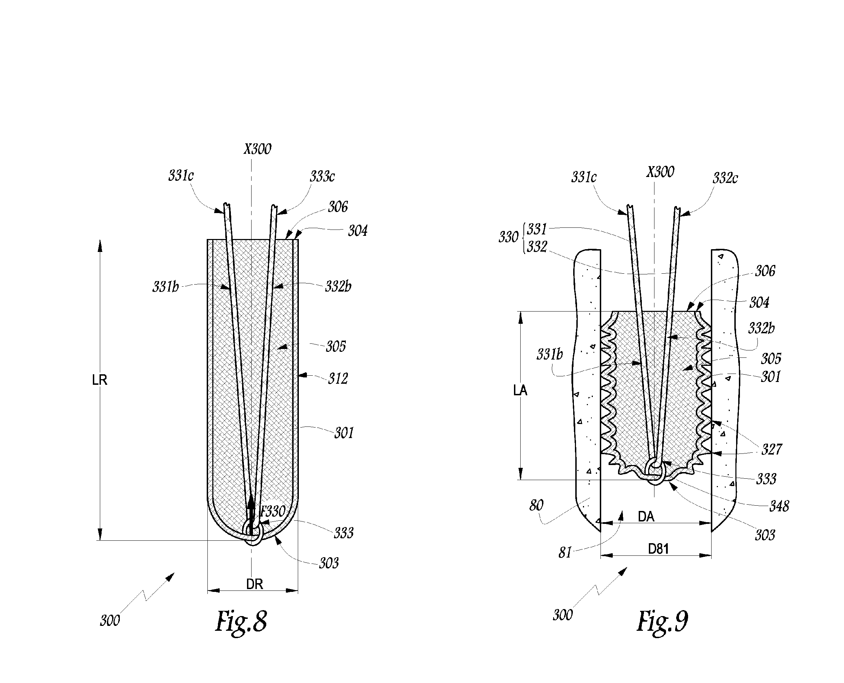

Sur les figures 8 et 9 est représenté le composant d'implant de suture 300.

Certains éléments constitutifs du composant 300 sont similaires aux éléments constitutifs du composant 100 du premier mode de réalisation, décrit plus haut, et portent la même référence augmentée de deux cent. Il s'agit de l'axe X300, du corps 301, de l'extrémité distale 303, de l'extrémité proximale 304, de l'ouverture 306, de l'espace intérieur 305, de la paroi 312, des dimensions DR et LR en configuration de repos, des dimensions DA et LA en configuration active, des aspérités 327, du fil 330, ainsi que des brins 331 et 332 qui sont reliés par un arc 333 et présentent des parties de brins 331 b, 332b, 331 c et 332c.

Le fil 330 ne traverse pas avec le corps 301 au niveau de l'extrémité distale 303 ou bien le long de la paroi 312. En revanche, un anneau 348 est attaché à l'extrémité distale 303. Cet anneau 348 est destiné à recevoir l'arc 333, selon une méthode d'implantation du fil 330 dans le composant 300 qui est plus rapide en comparaison avec le premier mode de réalisation. De préférence, l'anneau 348 est en matériau résorbable.

Ainsi, le fil 330 coopère avec le corps 301 du composant 300 par l'intermédiaire de l'arc 333 et de l'anneau 348. Une tension F330 peut être exercée sur l'extrémité distale 303 du corps 301 en manipulant le fil 330, déformant ainsi le corps 301 en configuration active.

Sur la figure 10 est représenté le composant d'implant de suture 400 en configuration de repos.

Certains éléments constitutifs du composant 400 sont similaires aux éléments constitutifs du composant 100 du premier mode de réalisation, décrit plus haut, et portent la même référence augmentée de trois cent. Il s'agit de l'axe X400, du corps 401, de l'extrémité distale 403, de l'extrémité proximale 404, de l'ouverture 406, de l'espace intérieur 405, de la paroi 412, des dimensions DR et LR en configuration de repos, du fil 430, ainsi que des brins 431 et 432 qui sont reliés par un arc 433 et présentent des parties de brins 431 a, 432a, 431 b, 432b, 431c et 432c.

L'extrémité distale 403 présente une ouverture 409, ainsi qu'un repli 403a qui s'étend dans l'espace intérieur 405 du corps 401 sous forme d'un bourrelet annulaire. Les brins 431 et 432 traversent non pas l'extrémité distale 403 mais la paroi 412, au niveau du repli 403a, puis forment l'arc 433. De ce fait, lors de la déformation du corps 401, l'extrémité distale 403 a tendance à se contracter en direction de l'axe X400.

Ainsi, le fil 430 coopère avec le corps 401 du composant 400 par l'intermédiaire des parties 431 a, 432a et de l'arc 433. Une tension F430 peut être exercée sur l'extrémité distale 403 du corps 401 en manipulant le fil 430, déformant ainsi le corps 401 en configuration active.

Sur la figure 11 est représenté le composant d'implant de suture 500 en configuration de repos.

Certains éléments constitutifs du composant 500 sont similaires aux éléments constitutifs du composant 100 du premier mode de réalisation, décrit plus haut, et portent la même référence augmentée de quatre cent. Il s'agit de l'axe X500, du corps 501, de l'extrémité distale 503, de l'extrémité proximale 504, de l'ouverture 506, de l'espace intérieur 505, de la paroi 512, des dimensions DR et LR en configuration de repos, du fil 530, ainsi que des brins 531 et 532 qui sont reliés par un arc 533 et présentent des parties de brins 531 a, 532a, 531 b, 532b, 531c et 532c.

L'extrémité distale 503 présente une ouverture 509, ainsi qu'un repli 503a qui s'étend dans l'espace intérieur 505 du corps 501 sous forme d'un bourrelet annulaire. Par ailleurs, l'extrémité proximale 504 présente un repli 504a qui s'étend dans l'espace intérieur 505 du corps 501 sous forme d'un bourrelet annulaire. Les brins 531 et 532 traversent la paroi 512, d'une part, au niveau du repli 503a pour former l'arc 533 et, d'autre part, au niveau du repli 504a pour permettre la manipulation des parties 531c et 532c. De ce fait, lors de la déformation du corps 501, l'extrémité distale 503 et l'extrémité proximale 504 ont tendance à se contracter en direction de l'axe X500. Autrement dit, les interférences sont formées dans la partie intermédiaire entre les extrémités 503 et 504, selon une localisation plus facile à contrôler en comparaison avec le composant 400 du quatrième mode de réalisation.

Le fil 530 coopère avec le corps 501 du composant 500 par l'intermédiaire des parties 531 a, 532a, 531 b, 532b et de l'arc 533. Une tension F530 peut être exercée sur l'extrémité distale 503 du corps 501 en manipulant le fil 530, déformant ainsi le corps 501 en configuration active.

Sur la figure 12 est représenté le composant d'implant de suture 600 en configuration de repos.

Certains éléments constitutifs du composant 600 sont similaires aux éléments constitutifs du composant 100 du premier mode de réalisation, décrit plus haut, et portent la même référence augmentée de cinq cent. Il s'agit de l'axe X600, du corps 601, de l'extrémité distale 603, de l'extrémité proximale 604, de l'ouverture 606, de l'espace intérieur 605, de la paroi 612, des dimensions DR et LR en configuration de repos, du fil 630, ainsi que des brins 631 et 632 qui sont reliés par un arc 633 et présentent des parties de brins 631a, 632a, 631 b, 632b, 631c et 632c.

L'extrémité distale 603 présente une ouverture 609, ainsi qu'un repli 603a qui s'étend dans l'espace intérieur 605 du corps 601 sous forme d'un bourrelet annulaire. Par ailleurs, l'extrémité proximale 604 présente un repli 604a qui s'étend dans l'espace intérieur 605 du corps 601 sous forme d'un bourrelet annulaire. Les brins 631 et 632 traversent la paroi 612, d'une part, au niveau du repli 603a pour former l'arc 633 et, d'autre part, au niveau du repli 604a pour permettre la manipulation des parties 631c et 632c.

Lors de la déformation du corps 601, l'extrémité distale 603 et l'extrémité proximale 604 ont tendance à se contracter en direction de l'axe X600, de même que les emplacements de la paroi tubulaire 612 qui coopèrent avec les parties 631 b et 632b du fil 630, de sorte que le corps 601 se déforme en « accordéon », par plissage et cintrage. Les interférences formées sur la paroi 612 sont réparties de façon homogène entre les extrémités 603 et 604. La déformation du composant 600 est analogue à celle du composant 100 du premier mode de réalisation, et est plus aisément contrôlable en comparaison avec les composants 400 et 500 des quatrième et cinquièmes modes de réalisation.

Par ailleurs, des monofilaments 608 sont répartis sur la paroi 612, du côté extérieur du corps 601. Ces monofilaments 608, ou d'autres fibres plus rigides, peuvent être tissées dans le corps 601 selon une répartition particulière, de sorte que lorsque le corps 601 est déformé, les monofilaments 608 se comportent comme des barbelures et renforcent l'adhérence du composant 600 dans la cavité 81.

En variante, les monofilaments 608 peuvent être disposés sur une partie seulement de la longueur LR du corps 601, selon la géométrie souhaitée en configuration active.

Le fil 630 coopère avec le corps 601 du composant 600 par l'intermédiaire des parties 631 a, 632a, 631 b, 632b et de l'arc 633. Une tension F630 peut être exercée sur l'extrémité distale 603 du corps 601 en manipulant le fil 630, déformant ainsi le corps 601 en configuration active.

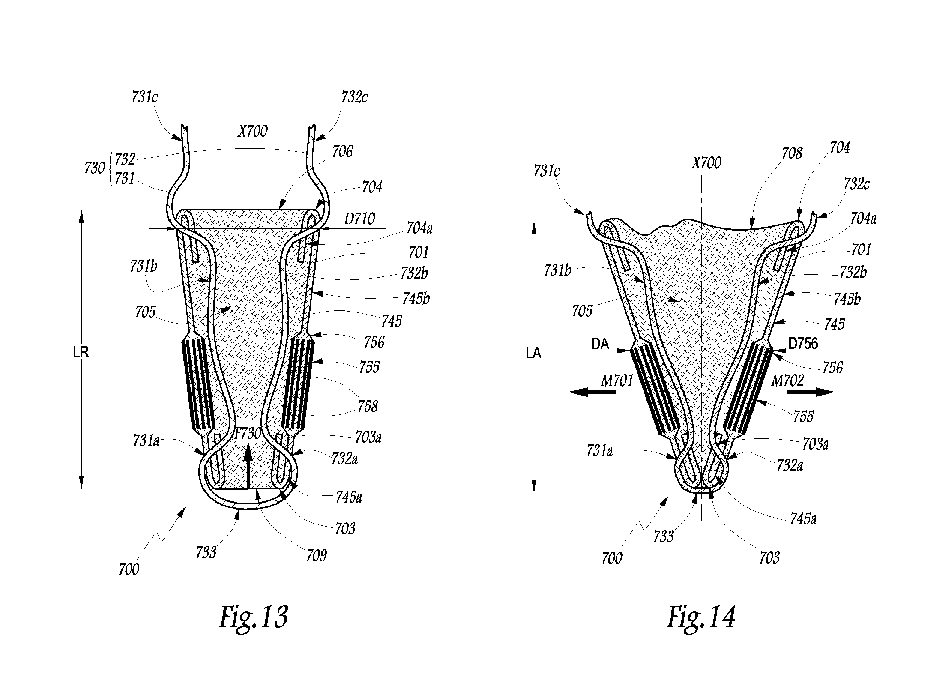

Sur les figures 13 et 14 est représenté le composant d'implant de suture 700.

Certains éléments constitutifs du composant 700 sont similaires aux éléments constitutifs du composant 100 du premier mode de réalisation, décrit plus haut, et portent la même référence augmentée de six cent. Il s'agit de l'axe X700, du corps 701, de l'extrémité distale 703, de l'extrémité proximale 704, de l'ouverture 706, de l'espace intérieur 705, de la paroi 712, des dimensions DR et LR en configuration de repos, de la longueur LA en configuration active, du fil 730, ainsi que des brins 731 et 732 qui sont reliés par un arc 733 et présentent des parties de brins 731 a, 732a, 731 b, 732b, 731c et 732c.

Le composant 700 est représenté, d'une part, en configuration de repos sur la figure 13 et, d'autre part, en configuration « semi-active » sur la figure 14, où le corps 701 n'est représenté que partiellement déformé par rapport à sa déformation attendue en configuration active. Le corps 701 présente une paroi 712 qui comprend une partie 745a du côté de l'extrémité distale 703, une partie intermédiaire 755 et une partie 745b du côté de l'extrémité proximale 704. Dans la configuration semi-active de la figure 14, les parties de paroi 745a et 755 sont en cours de déformation. En configuration active, la partie de paroi 745b est écrasée contre la paroi de la cavité osseuse 81 et subit une déformation de type « accordéon » analogue à celle des modes de réalisation précédents.

Par ailleurs, des monofilaments 758 sont positionnés dans la partie de paroi 755, par exemple tissées à l'intérieur du corps 701. Ces monofilaments 758 sont des fibres plus rigides que les fibres qui constituent le corps 701, de sorte que, lors de la déformation du corps 701, la partie 755 présente une résistance à la déformation supérieure aux parties 745a et 745b.

L'extrémité distale 703 présente une ouverture 709, ainsi qu'un repli 703a qui s'étend dans l'espace intérieur 705 du corps 701, contre la partie de paroi 745a, sous forme d'un bourrelet annulaire. Par ailleurs, l'extrémité proximale 704 présente un repli 704a qui s'étend dans l'espace intérieur 705 du corps 701, contre la partie de paroi 745a, sous forme d'un bourrelet annulaire. Les brins 731 et 732 traversent la paroi 712, d'une part, au niveau du repli 703a pour former l'arc 733 et, d'autre part, au niveau du repli 704a pour permettre la manipulation des parties 731c et 732c.

Lors de la déformation du corps 701, l'extrémité distale 703 et l'extrémité proximale 704 ont tendance à se contracter en direction de l'axe X700. La partie 745b se déforme en « accordéon », tandis que les parties 745a et 755 se déforment en « parapluie ». En fait, la partie 745a se contracte et l'ouverture distale 709 se referme, tandis que la partie 755 s'ouvre radialement de manière comparable à un parapluie, selon un déplacement qui est représenté par des flèches M701 et M702 sur la figure 14, en configuration semi-active. Ainsi, l'appui de la partie 755 contre la cavité osseuse 81 est réalisé sur le périmètre 756, qui présente un diamètre D756. En configuration active, le diamètre D756 est supérieur au diamètre DR et correspond au diamètre DA, qui est lui-même égal au diamètre D81 de la cavité 82.

En pratique, le composant 700 adhère donc à la cavité 81, d'une part, au niveau du périmètre 756 et, d'autre part, au niveau de la partie 745b de paroi 712 déformée en « accordéon ». La déformation en accordéon de la partie 745b autour de l'axe X700 forme plusieurs plis. Le fil 730 coopère avec le corps 701 du composant 700 par l'intermédiaire des parties 731 a, 732a, 731 b, 732b et de l'arc 733. Une tension F730 peut être exercée sur l'extrémité distale 703 du corps 701 en manipulant le fil 730, afin de déformer le corps 701 en configuration active.

En variante, les monofilaments 758 peuvent être disposés sur différentes parties de la paroi 745, avantageusement répartis sur la longueur LR du corps 701 selon la géométrie souhaitée en configuration active. Les monofilaments 758 peuvent être agencés de manière annulaire, axiale, ou une combinaison des deux. Par exemple, deux parties extrêmes de la paroi 745 peuvent comprendre des monofilaments 758, tandis que la partie intermédiaire ne comprend pas de monofilaments 758. Dans ce cas, le corps 701 se déforme comme un « double-parapluie ». Egalement, des renforts supplémentaires peuvent être intégrés dans le corps 701 pour former les « baleines » de parapluie.

De préférence, l'ouverture prévue pour recevoir le fil de suture est située du côté de l'extrémité proximale du corps, de sorte que le fil de suture pénètre dans l'espace intérieur du corps et coopère avec le corps au moins du côté de l'extrémité distale.

Toutefois, selon différentes variantes de réalisation de l'invention, le fil de suture peut être connecté uniquement à l'extrémité distale du corps, ou bien être cousu à travers la paroi du corps, ou être directement tissé dans le corps. En particulier, les passages multiples du fil de suture à l'intérieur et à l'extérieur des parois permettent d'accompagner l'action de cintrage, c'est-à-dire la déformation en accordéon. Le corps du composant peut présenter une extrémité distale fermée ou ouverte.

En variante, le composant d'implant de suture ne présente pas un corps tubulaire. En particulier, le corps peut présenter une paroi effilée de forme conique. Lorsque la paroi conique est plus réduite du côté de l'extrémité distale et qu'une tension est exercée sur cette extrémité distale, celle-ci est tirée vers l'espace intérieur du corps et la paroi conique est contrainte de s'étendre transversalement dans la cavité osseuse. Autrement dit, lorsque le composant est tissé ou tressé avec une forme conique, sa géométrie en configuration active peut être facilement maîtrisée.

Selon une variante particulière non représentée, le composant d'implant présente un mécanisme de type sangle (« cinch type mechanism » en anglais). De préférence, ce mécanisme est réalisé dans un matériau similaire au fil de suture, sans métal, et intégré dans le corps du composant d'implant lors de sa fabrication, par exemple par tissage. Une boucle de sangle s'étend depuis l'extrémité distale du corps, tandis que le fil de suture s'enroule autour de l'extrémité proximale du corps, puis traverse la boucle de sangle. Ainsi, le fil de suture est verrouillé dans le composant, avec ses extrémités libres disponibles pour que le chirurgien puisse former un noeud liant.

Selon une autre variante non représentée, un clip, une agrafe, un anneau ou un bouton peut être disposé dans le composant de sorte que le corps déformé puisse être verrouillé en configuration active.

Par ailleurs, la pointe de l'aiguille d'insertion du composant dans la cavité osseuse peut être configurée pour le poinçonnage ou le perçage. Ainsi, le chirurgien n'a pas besoin de pré-percer une cavité osseuse dans l'os, car la pointe est adaptée pour former directement cette cavité osseuse. De préférence, le composant présente alors une ouverture distale afin que la pointe de l'aiguille n'endommage pas le corps du composant.

En alternative, le dispositif d'implant de suture qui est à la disposition du chirurgien ne comporte pas d'aiguille d'insertion et/ou de manchon de compression. Dans ce cas, d'autres moyens de manipulation et d'insertion du composant d'implant de suture dans la cavité osseuse peuvent être mis en oeuvre. De préférence, ces moyens d'insertion sont amovibles et peuvent être retirés du composant en configuration active.

Selon une autre alternative, le composant peut être développé à l'aide d'un polymère, d'un ciment, ou d'un ballon gonflable, lequel est introduit dans le corps pour étendre le diamètre du corps jusqu'à atteindre le diamètre de la cavité osseuse. Ainsi, le composant est ancré contre la cavité par l'action d'une pression interne complémentaire de la déformation du corps. L'élément d'expansion peut appartenir à l'implant ou être retiré après expansion. L'élément d'expansion peut également être localisé pour favoriser une géométrie particulière en configuration active, telle qu'une forme de parapluie.

Quel que soit le mode de réalisation du composant d'implant de suture selon l'invention, la paroi du corps de l'implant est déformable en accordéon autour de son axe longitudinal. Dans ce cas, plusieurs plis sont formés sur au moins une partie de cette paroi, autour de l'axe longitudinal.

En outre, les caractéristiques techniques des différents modes de réalisation peuvent être, en totalité ou pour certaines d'entre elles, combinées entre elles. Ainsi, le composant d'implant de suture peut être adapté aux besoins particuliers du chirurgien et/ou du patient dans le cadre de l'application visée, et présente de nombreux avantages en comparaison avec les dispositifs existants.

## Revendications

1. Composant d'implant de suture (100 ; 200 ; 300 ; 400 ; 500 ; 600 ; 700), comprenant un corps (101 ; 201 ; 301 ; 401 ; 501 ; 601 ; 701) qui s'étend suivant un axe longitudinal (X100 ; X200 ; X300 ; X400 ; X500 ; X600 ; X700) et qui inclut au moins une ouverture (106 ; 206 ; 306 ; 406 ; 506 ; 606 ; 706) apte à recevoir un fil de suture (130 ; 230 ; 330 ; 430 ; 530 ; 630 ; 730), de sorte que le fil de suture coopérant avec le corps forme sensiblement un U ou un V, le corps (101 ; 201 ; 301 ; 401 ; 501 ; 601 ; 701) étant déformable, sous l'effet d'une tension (F130 ; F230 ; F330 ; F430 ; F530 ; F630 ; F730) exercée par le fil de suture (130 ; 230 ; 330 ; 430 ; 530 ; 630 ; 730) au moins sur une extrémité distale (103 ; 203 ; 303 ; 403 ; 503 ; 603 ; 703) du corps, entre :
- une configuration de repos dans laquelle le corps (101 ; 201 ; 301 ; 401 ; 501 ; 601 ; 701) présente une première valeur de dimension longitudinale (LR) et une première valeur de dimension transversale (DR), et
- une configuration active dans laquelle le corps (101 ; 201 ; 301 ; 401 ; 501 ; 601 ; 701) présente une deuxième valeur de dimension longitudinale (LA) qui est inférieure à la première valeur de dimension longitudinale (LR) et une deuxième valeur de dimension transversale (DA) qui est supérieure à la première valeur de dimension transversale (DR),
**caractérisé en ce que** le corps (101 ; 201 ; 301 ; 401 ; 501 ; 601 ; 701) présente une paroi (112 ; 212 ; 312 ; 412 ; 512 ; 612 ; 712) qui est agencée autour de l'axe longitudinal (X100 ; X200 ; X300 ; X400 ; X500 ; X600 ; X700) et qui, lorsque le corps passe de sa configuration de repos à sa configuration active, est déformable en accordéon.

2. Composant d'implant de suture selon la revendication 1, **caractérisé en ce que** la paroi (112 ; 212 ; 312 ; 412 ; 512 ; 612 ; 712) agencée autour de l'axe longitudinal (X100 ; X200 ; X300 ; X400 ; X500 ; X600 ; X700) est déformable en accordéon par plissage, cintrage, écrasement et/ou contraction, lorsque le corps (101 ; 201 ; 301 ; 401 ; 501 ; 601 ; 701) passe de sa configuration de repos à sa configuration active.

3. Composant d'implant de suture selon l'une des revendications précédentes, **caractérisé en ce que** la paroi (112 ; 212 ; 312 ; 412 ; 512 ; 612 ; 712) est tubulaire ou conique.

4. Composant d'implant de suture selon l'une des revendications précédentes, **caractérisé en ce que**, lorsque le corps (101 ; 201 ; 301 ; 401 ; 501 ; 601 ; 701) est en configuration active, la paroi (112 ; 212 ; 312 ; 412 ; 512 ; 612 ; 712) déformée en accordéon autour de l'axe longitudinal (X100 ; X200 ; X300 ; X400 ; X500 ; X600 ; X700) forme plusieurs plis.

5. Composant d'implant de suture selon l'une des revendications précédentes, **caractérisé en ce que** le corps (101 ; 201 ; 301 ; 401 ; 501 ; 601 ; 701) est tissé ou tressé avec des fibres en matériau biocompatible, de préférence en matériau résorbable, ces fibres étant fabriquées avec le ou les matériaux suivants : polyester, polyéthylène, polyhydroxyalkanoate ou une combinaison d'au moins certains d'entre eux.

6. Composant d'implant de suture selon l'une des revendications précédentes, **caractérisé en ce que** le corps (101 ; 201 ; 301 ; 401 ; 501 ; 601 ; 701) présente au moins deux types de fibres, ayant des diamètres respectifs différents.

7. Composant d'implant de suture (200) selon l'une des revendications précédentes, **caractérisé en ce que** le fil de suture (230) comprend deux brins (231, 232) qui traversent l'extrémité distale (203) du corps (201) et sont reliés par un arc (233) pourvu d'un noeud qui est situé à l'extérieur de l'extrémité distale (203) du corps (201).

8. Composant d'implant de suture (600) selon l'une des revendications précédentes, **caractérisé en ce que** des monofilaments (608) sont répartis sur une paroi (612) du corps (601), du côté extérieur, sur au moins une partie de la longueur du corps.

9. Composant d'implant de suture (700) selon l'une des revendications précédentes, **caractérisé en ce que** des monofilaments (758) sont intégrés dans le corps (701) sur au moins une partie (755) de la longueur du corps.

10. Composant d'implant de suture (200) selon l'une des revendications précédentes, **caractérisé en ce que** le corps (201) présente un anneau de rigidification (207) positionné à une extrémité proximale (204).

11. Composant d'implant de suture (100 ; 200 ; 300) selon l'une des revendications précédentes, **caractérisé en ce que** le corps (101 ; 201 ; 301) présente une extrémité proximale (104 ; 204 ; 304) munie d'une ouverture (106 ; 206 ; 306), tandis que l'extrémité distale (103 ; 203 ; 303) est fermée.

12. Composant d'implant de suture selon l'une des revendications précédentes, **caractérisé en ce que** le corps (101 ; 201 ; 301 ; 401 ; 501 ; 601 ; 701) comprend un élément de verrouillage en configuration active, par exemple un clip, une agrafe, un anneau ou un bouton disposé dans le corps.

13. Composant d'implant de suture selon l'une des revendications précédentes, **caractérisé en ce que** le corps (101 ; 201 ; 301 ; 401 ; 501 ; 601 ; 701) comprend un mécanisme de sangle disposé à son extrémité distale (103 ; 203 ; 303 ; 403 ; 503 ; 603 ; 703), adapté pour recevoir et verrouiller le fil de suture (130 ; 230 ; 330 ; 430 ; 530 ; 630 ; 730), le mécanisme de sangle étant de préférence réalisé dans un matériau similaire au fil de suture (130 ; 230 ; 330 ; 430 ; 530 ; 630 ; 730) et intégré dans le corps (101 ; 201 ; 301 ; 401 ; 501 ; 601 ; 701) lors de la fabrication du corps, par exemple par tissage.

14. Dispositif d'implant de suture (1), **caractérisé en ce qu'**il comprend :
- un composant d'implant de suture (100 ; 200 ; 300 ; 400 ; 500 ; 600 ; 700) selon l'une des revendications précédentes,
- un fil de suture (130 ; 230 ; 330 ; 430 ; 530 ; 630 ; 730), et
- des moyens (131 a, 132a, 131 b, 132b, 133 ; 231 a, 231 b, 233 ; 333, 348 ; 431 a, 432a, 433 ; 531 a, 532a, 531 b, 532b, 533 ; 631 a, 632a, 631 b, 632b, 633 ; 731 a, 732a, 731 b, 732b, 733) de coopération du fil de suture avec le corps (101 ; 201 ; 301 ; 401 ; 501 ; 601 ; 701) du composant, situés au moins du côté de l'extrémité distale (103 ; 203 ; 303 ; 403 ; 503 ; 603 ; 703) du corps.

15. Dispositif d'implant de suture (1) selon la revendication précédente, **caractérisé en ce qu'**il comprend également des moyens (50, 60) amovibles d'insertion du composant d'implant de suture (100 ; 200 ; 300 ; 400 ; 500 ; 600 ; 700) dans une cavité osseuse (81), ces moyens incluant :
- une aiguille (60) adaptée pour maintenir, dans la configuration de repos, la dimension longitudinale (LR) et la dimension transversale (DR) du corps (101), cette aiguille étant une tige agencée dans l'espace intérieur (105) du corps à travers l'ouverture (106) située à une extrémité proximale (104) du corps, et/ou
- un manchon tubulaire (50) adapté pour maintenir en position l'extrémité proximale (104) du corps lors de la déformation du corps (101) entre la configuration de repos et la configuration active, ce manchon étant agencé coaxialement au corps en appui contre une extrémité proximale du corps.

16. Dispositif d'implant de suture (1) selon la revendication précédente, **caractérisé en ce que** l'aiguille (60) comprend une pointe (63) de poinçonnage ou de perçage, laquelle est notamment configurée pour former une cavité osseuse (81) dans un os (80) pour l'insertion du composant d'implant de suture (100 ; 200 ; 300 ; 400 ; 500 ; 600 ; 700) dans cet os.
